(19) Europäisches Patentamt
European Patent Office
Office européen des brevets

(11) **EP 2 207 432 B1**

(12) # EUROPEAN PATENT SPECIFICATION

(45) Date of publication and mention
of the grant of the patent:
**08.07.2015 Bulletin 2015/28**

(21) Application number: **08804072.0**

(22) Date of filing: **11.09.2008**

(51) Int Cl.:
*A23K 1/16* (2006.01)        *A23K 1/18* (2006.01)

(86) International application number:
**PCT/EP2008/062107**

(87) International publication number:
**WO 2009/034151 (19.03.2009 Gazette 2009/12)**

(54) **TREATMENT OF PIGS FOR REDUCING THE FEED CONVERSION RATIO OR INCREASING THE GROWTH RATE**

BEHANDLUNG VON SCHWEINEN ZUR ERNIEDRIGUNG DER FUTTERVERWERTUNGSRATE BZW. STEIGERUNG DER WACHSTUMSRATE

TRAITEMENT DE PORCS POUR RÉDUIRE L'INDICE DE CONSOMMATION OU AUGMENTER LA VITESSE DE CROISSANCE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **12.09.2007 PCT/EP2007/059596**

(43) Date of publication of application:
**21.07.2010 Bulletin 2010/29**

(73) Proprietor: **Taminco
9000 Gent (BE)**

(72) Inventors:
• **KALMAR, Isabelle**
**B-9000 Gent (BE)**
• **JANSSENS, Geert**
**B-9270 Laarne (BE)**
• **ROOSE, Peter**
**B-9831 Sint-Martens-Latem (BE)**
• **VANNESTE, Piet**
**B-9400 Denderwindeke (BE)**

(74) Representative: **Van Reet, Joseph et al
Gevers
Intellectual Property House
Holidaystraat 5
1831 Diegem (BE)**

(56) References cited:
**WO-A-99/04784      WO-A-2007/107184
US-A- 4 631 189**

• **M. Eklund ET AL: "Potential nutritional and physiological functions of betaine in livestock", Nutrition Research Reviews, vol. 18, no. 01, 1 June 2005 (2005-06-01), page 31, XP055169577, ISSN: 0954-4224, DOI: 10.1079/NRR200493**
• **"Metabolic, osmoregulatory and nutritional functions of betaine in monogastric animals", , 1 January 2009 (2009-01-01), XP055169580, Retrieved from the Internet: URL:http://www.ajas.info/upload/pdf/22-191 .pdf[retrieved on 2015-02-13]**

**Description**

The present invention relates to a method for the non-therapeutic treatment of pigs for the purpose of reducing the conversion ratio of the feed used to feed the pigs and/or for increasing the growth rate of the pigs by means of a particular agent, and to a feed for pigs containing an amount of that agent.

In the pork industry, improvements and developments have been made essentially in the breeding technique for phyletic lines of pigs and in the rearing technique for increasing the growth rate of grower and finisher pigs. Much emphasis is put on the growth rate and the feed conversion ratio in the method of rearing pigs. A high-calorie feed enables to achieve a lower feed conversion ratio, i.e. a lower amount of feed is required to produce a certain amount of pork meat. However, a further reduction of the feed conversion or a further increase of the growth rate is always desired to reduce the production costs.

In practice, it is indeed of high economical importance to be able to decrease the feed conversion ratio, i.e. the amount of feed required for 1 kg of productivity, being either gain in body weight or production of weaned piglets, without having to use a (more expensive) feed having a higher energy or nutrient value.

An object of the present invention is therefore to provide a new treatment of pigs which enables to reduce the conversion ratio of the feed used to feed the pigs, i.e. which enables in the case of weaned piglets/fattening pigs to reduce the amount of feed required to produce a certain amount of pork meat or which enables in the case of sows to reduce the amount of feed required to produce an amount of piglets.

In a first aspect, the present invention concerns a method for the non-therapeutic treatment of pigs for the purpose of reducing the conversion ratio of the feed used to feed the pigs and/or for increasing the growth rate of the pigs, which treatment comprises orally administering a glycine compound to the pigs, which glycine compound is selected from N,N-dimethylglycine or a salt thereof.

In a second aspect, the present invention concerns a feed for pigs which comprises at least 0.001 % by weight of said glycine compound, being selected from N,N-dimethylglycine or a salt thereof.

N,N-Dimethylglycine (DMG) was first detected in 1938 as part of the active molecule pangamic acid (6-O-(dimethylaminoacetyl)-D-gluconic acid, PA) (Krebs E.T., Beord N.H., Malin R. Int. Red. Med. 164, 18 (1954)). The original report by Krebs declares that PA is always found together with the known B-vitamins, this fact together with the assigned biological functions were taken as reasons to account PA as vitamin $B_{15}$, although no disease state can as yet be attributed exclusively to a deficiency of the substance. Russian researchers reported that calcium pangamate could have a positive effect on the performance of athletes and on the cardiovascular and liver function. Further Russian studies showed that the pangamate formula has some influence on the restore of the immune system of guinea pigs and rats which were subjected to high intensity X-rays (Nizametidinova, G. Reports of the Kazan Veterinary Institute 112, 100-104 (1972)).

US-A-3 907 869 defines the component calcium pangamate as the ester of dimethylglycine and calcium gluconate and describes as advantages of PA the capability of enhancing oxidative metabolism in cells and tissues and eliminating the phenomena of hypoxia, as well as promoting lipid metabolism and acting as detoxicant.

As described in the article of Roger V. Kendall and John W. Lawson, "Recent Findings on N, N-Dimethylglycine (DMG): A new Nutrient for the New Millenium" (2000), calcium pangamate would not be stable under normal digestive processes and would rapidly hydrolyze to DMG when given after oral administration. When describing the known effects of DMG in their article, these authors thus considered DMG as the active component of calcium pangamate.

WO 99/04784 discloses the use of prophylactics, especially betaines or related products, to enhance the resistance of animals to infection by mycoplasma or other organisms causing similar damage. In the only example described in this patent application the feed of male grower - finisher pigs was supplemented with 0.125 % betaine and a significant reduction of the lung area damaged by mycoplasmal infection was achieved. It was also found that the use of betaine resulted in improvements in the growth rate and other performance characteristics, such as the feed conversion ratio, of growing pigs when exposed to mycoplasmal infections. WO 99/04784 is thus primarily concerned with the therapeutic (prophylactic) use of betaine. DMG is also mentioned but only amongst the other prophylactics which might be suitable for enhancing the resistance of the pigs against mycoplasmal infections. These other prophylatics are only shortly enumerated but were not tested in any way.

In contrast to the method disclosed in WO 99/04784, the method according to the present invention is a method for the non-therapeutic treatment of pigs so that the feed conversion ratio can also be significantly improved for healthy pigs.

Kalmar I.D. et al. disclose in the abstract of their presentation on the 9th ESVCN Congress, 22nd - 24th September 2005, Grugliaslco (TO), Italy, "Effect of dimethylglycine on performance and ascites-related parameters in broiler chickens", that DMG has a positive effect on feed conversion rate in growing broilers. Poultry and pigs have however a different physiology so that effects on poultry cannot be extrapolated as such to pigs. For example, the lungs of birds are firm and fixed into the thoracic cavity and do not expand and contract with each breath as mammalian lungs; moreover avian lungs are smaller as a percentage of body weight than those of mammals. These specific anatomical and physiological traits of the avian respiratory system makes birds and more in particular fast growing meat type chickens highly prone to develop pulmonary hypertension syndrome (broiler ascites syndrome), which is considered one of the most important causes of mortality in broilers. The experiments described in the abstract of Kalmar et al. were in the first place intended to evaluate the possible effects of DMG on ascites. Furthermore, according to Kalmar et al. DMG supplementation decreased the feed conversion rate in broilers, as described in the present specification for pigs, but it had not an important impact on body weight gain, in contrast to the current invention relating to pigs. The observed differences in effects and magnitude of effects of DMG between broilers and pigs support the hypothesis of multiple ways of action of DMG, manifesting differently in birds and mammals as determined by the specific anatomy, physiology and rearing conditions of the target species.

Fekete (Fekete S., Hegedüs M., Sós E. Magyar Állatorvosok Lapja 34(5), 311-314 (1978)) has also done tests to verify whether it might be possible to reduce the feed conversion rate of broilers not by supplementing the diet with DMG but instead with pangamic acid (vitamin $B_{15}$), which is an ester of D-gluconic acid with DMG. Fekete observed a reduction of the body weight gain whilst the feed conversion rate was not affected by the pangamic acid.

In accordance with the present invention it has been found that by orally administering DMG to pigs, the feed conversion ratio could be substantially reduced for pigs, more particularly for perfectly healthy pigs, and this both for male and female pigs. In contrast to broilers, the growth rate of a population of male and female pigs could also be increased by DMG supplementation. This was mainly due to a major increase of the growth rate of male pigs since the growth rate of female pigs even decreased.

As mentioned hereabove, the present invention is concerned with a method for the non-therapeutic treatment of pigs for the purpose of reducing the conversion ratio of the feed used to feed the pigs, and/or for increasing the growth rate of the pigs, by orally administering N,N-dimethylglycine (DMG) or a salt thereof to the pigs, and to a feed for pigs comprising N,N-dimethylglycine (DMG) or the salt thereof.

The invention is applicable to any type of commercial pig operation but is primarily concerned with growing pig operations. In a commercial pig production operation the herd is typically under substantial stress. As is well known, normal industry growing conditions include substantial density in the enclosure, for example, a density on the order of about 1.1 $m^2$ per piglet until 25 kg. Further, the ventilation in such commercial growing operations is often not a precisely controlled operation and the determination of appropriate ventilation including both heating and cooling is a very subjective operation. Further, the life span for slaughter pigs is around 6 months whilst sows are usually removed after 3 rounds so that the whole operation from birth to market in conditions under which growth/reproduction is achieved is very stressful to the herd. Moreover, to aggravate the problem, growers will typically push the limits of recommended industry conditions which simply increases the stress on the herd.

Due to these high performance conditions, the occurrence level of metabolic problems, especially in piglets, is already quite high in practice and limits the development of new feeds or production methods which causes even more metabolic or oxidative stress. A higher oxidative stress is for example obtained when the feed compositions contain more unsaturated fatty acids, for example more than 3 % by weight or more than 4 or even 5 % by weight of the feed, whilst a higher metabolic stress is obtained when the pigs are made to take up more calories to increase performance. The fatty acids contained in the feed compositions are either free fatty acids or fatty acids bound for example in di- or triglycerides.

Nowadays feed compositions for pigs are more and more supplemented with fats from vegetal sources, as a cheaper alternative to fish oil and a safer alternative to animal fat. Hence, because of growing customer demand for use of vegetarian diets in meat production in order to avoid potential hazards peculiar to animal fat such as PCB, dioxin or BSE contamination. A second reason is to increase the amount of PUFA's (poly unsaturated fatty acids) in the meat, improving the nutritional value of pork without compromising the total energy value of the feed for the animals. As a direct effect of this increased level of vegetal fat in the feed the dietary induced oxidative stress increases, which leads to genotoxicity (DNA damage) and tissue damage.

According to the present invention, it has been found that administration of appropriate and effective amounts of a glycine compound selected from N,N-dimethylglycine (DMG) or a salt thereof, to pigs being grown under commercial growing operations enables to reduce the feed conversion ratio and thus enables to use the feed more efficiently. In contrast to the results obtained with betaine in WO 99/04784, these effects are not due to a reduction of mycoplasmal pneumonia infections but may possibly be explained by a reduction of the oxidative stress by means of DMG. Further tests will have to demonstrate whether the effect of DMG on the feed conversion ratio is indeed at least partially due to a reduction of the oxidative stress.

N,N-dimethylglycine (DMG) or the salt thereof can be dosed in the drinking water of the pigs. Most preferably, N,N-dimethylglycine (DMG) or the salt thereof is administered via the feed of the pigs.

The basal diet to which the N,N-dimethylglycine (DMG) or the salt thereof is added can be any typical pig diet meeting the nutritive needs depending on production purpose: growth, gestation or lactation. A conventional diet includes selections among various protein, carbohydrate, vitamin and mineral sources and will generally contain about 8-26 % by weight crude protein, 2-12 % by weight crude fat and 2-13 % by weight crude fiber. Pig diets for growth preferably have a crude protein content of at least 18%, 18%, 17%, 14% and 12% by weight for slaughter or fattening pigs of 1-5 kg, 5-10 kg, 10-20 kg, 20-50 kg and 50-110 kg respectively. Pig diets for growth have further preferably a crude fat content between 4 and 9 % by weight, a crude fibre content of less than 5.5 % by weight. Diets for adult, breeding boars and sows have preferably a crude fat content between 3.5 and 11% by weight and a crude protein content of at least 13% by weight. Diets for lactating sows contain preferably at least 13% crude protein by weight.

The primary component is generally grain and processed grain by-products which supply carbohydrates and some protein. Protein meals from soybeans, alfalfa, corn gluten, cottonseed, sunflowers and other plants are often used to supply additional protein to the diet, as are animal by-products. Pig feed compositions are generally supplemented with various vitamins and minerals (usually in the form of a premix to which the glycine compound can also be added), and molasses are added to improve palatability and to increase or balance energy levels. They have generally a moisture content of less than 15 % by weight and preferably of less than 14 % by weight. General reference is made to National Research Council, Nutrient Requirements of Swine (Ninth Revised Edition, 1988).

The feed according to the invention has usually a metabolizable energy (ME) value of at least 12.4 MJ/kg, and at the most 16.7 MJ/kg, for slaughter (fattening) pigs, preferably at least 13.5 MJ/kg for slaughter (fattening) pigs between 1 and 5 kg, at least 13.6 MJ/kg for slaughter pigs between 5 and 50 kg, and at least 13.7 MJ/kg for slaughter pigs between 50 and 110 kg. For developing boars and gilts, the feed has usually a metabolizable energy value of at least 13.0 MJ/kg, preferably at least 13.6 MJ/kg and at the most 14.0 MJ/kg, for gestating sows the feed has usually an ME value of at least 10 MJ/kg and for lactating sows an ME value of at least 13.0 MJ/kg, preferably at least 13.4 MJ/kg and at the most 15.7 MJ/kg. By means of such a high-calorie feed, high growth rates and low feed conversion ratio's can be achieved. The N,N-dimethylglycine (DMG) or the salt thereof is preferably administered at least during a period to the pigs which are selected and raised in such a manner that over this period the actual feed conversion ratio is smaller than 4, preferably smaller than 3.5 and more preferably smaller 3. The N,N-dimethylglycine (DMG) or the salt thereof is preferably administered over a period of 7 days or longer, preferably over a period of 14 days or longer.

The metabolizable energy as referred to herein is obtained by subtracting the amount of energy as discharged in faeces and urine (waste calorie) from the amount of total energy of the intake feed (total calorie), and ordinary calorimetry may be applied to each feed composition to actually obtain the metabolizable energy of the composition. In general, the metabolizable energy value of feed for chickens is obtained on the basis of known tables of calorie components for it, and such known tables may be employed herein to obtain the metabolizable energy in question. The apparent metabolizable energy value can more particularly be calculated by means of the following formula:

$$\text{AME (MJ/kg)} = 19.7\ \text{CP} + 32.2\ \text{EE} + 18.2\ \text{St} + 15.9\ \text{Su} + 0.5\ \text{Re}$$

Wherein: AME = apparent metabolizable energy (MJ/kg)

CP = crude protein (%)

EE = ether extract (= crude fat) (%)

St = starch (%)

Su = sugars (%)

Re = Residue

(see Noblet J, van Milgen J, (2004) Energy value of pig feeds: Effect of pig body weight and energy evaluation system. J. Anim Sci.82:E229-E238).L

The standard (AOAC International) analysis methods are:

- dry matter: drying
- crude protein: Kjeldahl method
- crude ash: incineration
- crude fat: ether extraction (Sohxlet method)
- crude fibre: Fibertec analysis
- starch and sugars: Luff-Schoorl analysis (polarimetry).

(see Official Methods of Analysis of AOAC International, 16th ed. The Association of Official Analytical Chemists, Arlington, VA).

In a preferred embodiment of the invention, the preferred additive range of N,N-dimethylglycine (DMG) or the salt thereof in the finished feed is about 0.001 to 0.5 % by dry weight, preferably about 0.005 to 0.1 % by dry weight, and more preferably about 0.01 and 0.08 % by dry weight. There is no evidence that use of the higher amounts would cause any toxicity problems in treated pigs; however, the considerations of cost may become significant.

## Experimental results

The setup of the experiments described below is to assess the influence of DMG on the performance in model species for pigs: mortality, feed conversion ratio (FCR), daily growth (dG), body weight (BW).

## Methodology and materials

To examine all the effects associated with the supplementation of dimethylglycine (DMG) in pig feed, a test was setup with pigs of Rattlerow Seghers origin as model species. In this feeding trial 60 weaned piglets of even sex distribution were kept for 42 days. Teeth of piglets were clipped and male piglets were neutered during the first week after birth. At the beginning of the test the piglets were ad random divided per sex in 20 pens with 3 piglets per pen, reckoning even weight distribution at pen level. The piglets were fed with one type of feed from the first day on till the end of the test period. The type of feed was attributed on a random basis to the different housings. The control feed was based on a commercial piglet feed enriched with 5% corn oil to increase the oxidative stress in the feed. The feed had the following composition: 91 % dry matter, 5 % ash, 18 % crude protein, 8% crude fat, 4% crude fibre and 56% other carbohydrate. It has a (calculated) metabolizable energy value of about 13.9 MJ/kg.

In the second type of feed 500mg of DMG was added per kilogram to the same composition of feed as described for the control feed. Over the entire test period, the pigs consumed on average about 290 mg DMG per piglet per day. The housing of the piglets consisted out of slatted floor pig pens of 1.05 m x 1.7 m with a total surface of 1.785 $m^2$. Feed and water were ad libitum available at all times.

Each piglet was weighted after overnight fasts at 4 times, namely at days 0, 14, 29 and 42. The daily growth for the total growth period (dG) as well as daily growth during the three periods (between the start and the fourteenth day (dGI), between the fourteenth and the twenty-ninth day (dGII) and between the twenty-ninth day and the forty-second day (dGIII)) was calculated for each animal. The feed intake for each pen was also measured during these periods. The feed conversion ratio was calculated for each of the pens for both the growth period excluding the first two weeks (FRCII+III) and for the total growth period (FRC). This feed conversion ratio was calculated by dividing the total feed consumption by the weight gain of the piglets during the entire experiment.

## Results

Starting body weight was not different between both experimental groups. All of the animals remained healthy during the experiment; more specifically, none of the animals showed clinical signs of infectious or non-infectious respiratory, intestinal or skin disease or developed leg problems. End body weight and daily growth of the piglets (barrows and gilts together) were numerically, but not significantly, improved through DMG supplementation (improvement = 1.9 % and 3.5 % respectively, P-values > 0.05). However, feed conversion ratio was significantly lower in the DMG supplemented groups (1.87) compared to the control groups (1.98) (improvement = 5.9 % P-value = 0.044). This improvement in feed conversion ratio was even higher in the growth period excluding the first two weeks (improvement 7.6 %, P-value = 0.004 %). (Table 1).

**Table 1:** Effects of DMG supplementation (500 mg/kg) in piglet feed on performance in piglets (barrows and gilts) (mean $\pm$ SD)

|  |  | DMG | Control | DMG-effect | P-value |
|---|---|---|---|---|---|
| Starting body weight | (kg) | 8.2 $\pm$ 0.25 | 8.2 $\pm$ 0.24 |  | 0.675 |
| End body weight | (kg) | 21.4 $\pm$ 2.46 | 21.0 $\pm$ 1.83 | + 1.9 % | 0.690 |
| Daily growth (dG) | (g/d) | 314 $\pm$ 53 | 303 $\pm$ 44 | +3.5 % | 0.637 |
| dGI | (g/d) | 94 $\pm$ 58 | 102 $\pm$ 37 | -7.0 % | 0.747 |
| dGII | (g/d) | 365 $\pm$ 65 | 338 $\pm$ 123 | + 8.1 % | 0.542 |
| dGIII | (g/d) | 491 $\pm$ 118 | 481 $\pm$ 194 | + 2.1 % | 0.891 |
| FCR |  | 1.87 $\pm$ 0.14 | 1.98 $\pm$ 0.05 | **-5.9 %** | **0.044** |
| FCRII+III |  | 1.72 $\pm$ 0.10 | 1.87 $\pm$ 0.04 | **-7.6 %** | **0.004** |

DMG effects on technical performance were dependent on gender. Overall, the effects were more prominent in barrows compared to gilts.

DMG supplementation in barrows resulted in a significantly higher end body weight (improvement = + 10.7 %, P-value = 0.038), a significantly higher growth rate (improvement = + 18.0, P-value = 0.036) and a significantly lower feed conversion ratio (improvement = 8.4 %, P-value = 0.019 %). Whereas gilts fed the DMG supplemented diet had a numerically, but not significantly, lower end body weight and growth rate, even though a significant lower feed conversion ratio during the growth period excluding the first two weeks (FCRII+III) (improvement = 8.9%, P-value = 0.012) (table 2).

**Table 2:** Gender differences in technical performance of piglets fed a control diet compared to the same diet supplemented with DMG (500 ppm) (mean ± SD)

| | DMG | Control | DMG-effect | P-value |
|---|---|---|---|---|
| **End BW** | | | | |
| Barrow | 23.4 ± 0.86 | 21.1 ± 1.84 | **+ 10.7 %** | **0.038** |
| Gilt | 19.3 ± 1.64 | 20.8 ± 2.03 | - 7.0 % | 0.245 |
| **dG** | | | | |
| Barrow | 356 ± 20 | 302 ± 44 | **+ 18.0 %** | **0.036** |
| Gilt | 271 ± 39 | 305 ± 48 | -11.0 % | 0.261 |
| **FCR** | | | | |
| Barrow | 1.80 ± 0.09 | 1.96 ± 0.06 | **- 8.4 %** | **0.019** |
| Gilt | 1.93 ± 0.16 | 2.01 ± 0.03 | - 3.6 % | 0.420 |
| **FCRII+III** | | | | |
| Barrow | 1.71 ± 0.12 | 1.83 ± 0.07 | - 6.2 % | 0.136 |
| Gilt | 1.74 ± 0.09 | 1.91 ± 0.04 | **- 8.9 %** | **0.012** |

In summary, it was found that the supplementation of 500 mg per kg feed of DMG, has a beneficial effect on technical performance in pigs. DMG supplementation has a distinct influence on the feed conversion ratio, an important economic parameter in the cultivation of pigs in general. Barrows profited more from DMG supplementation compared to gilts. Herewith, feed efficiency improved significantly with 8.4 % in barrows but improved only numerically with 3.6 % in gilts. However, when excluding the first two weeks after weaning, feed conversion ratio of gilts improved significantly with 8.9 % when DMG is supplemented to the diet. Daily growth and ending bodyweight was significantly enhanced by DMG supplementation in barrows with 18.0 % and 10.7 % respectively. Hence, dietary DMG supplementation has been shown to significantly and relevantly improve both growth and feed efficiency in barrows. Further, DMG supplementation has been demonstrated to significantly improve feed efficiency in gilts.

**Claims**

1. A method for the non-therapeutic treatment of pigs for the purpose of reducing the conversion ratio of the feed used to feed the pigs and/or for increasing the growth rate of the pigs, which treatment comprises orally administering at least one glycine compound to the pigs, which glycine compound is selected from N,N-dimethylglycine (DMG) or a salt thereof.

2. The method according to claim 1, wherein the glycine compound is administered via the drinking water of the pigs.

3. The method according to claim 1 or 2, wherein the glycine compound is administered via said feed.

4. The method according to any one of the claims 1 to -3, wherein the glycine compound is administered in an amount of between 0.001 and 0.5 % by dry weight of said feed, preferably in an amount of between 0.005 and 0.1 % by dry weight of said feed and more preferably in an amount of between 0.01 and 0.08 % by dry weight of said feed.

5. The method according to any one of the claims 1 to 4, wherein the glycine compound is orally administered to male pigs.

**6.** The method according to any one of the claims 1 to 5, wherein the pigs comprise weaned piglets or fattening pigs, the glycine compound being preferably included in the feed which is fed to the pigs.

**7.** The method according to claim 6, **characterised in that** said pigs are between 21 days and 7 months of age.

**8.** The method according to any one of the claims 1 to 4, wherein the pigs comprise gestating or lactating sows, the conversion ratio of the feed being determined taking into account the weight of the sows and, for lactating sows, the weight of the sows and their piglets, and the glycine compound being preferably included in a parturition feed or in a lactation feed which is fed to the sows.

**9.** Use of a glycine compound for reducing the conversion ratio of feed used to feed pigs and/or for increasing the growth rate of pigs, which glycine compound is selected from N,N-dimethylglycine (DMG) or a salt thereof.

**10.** Use according to claim 9, wherein said glycine compound is used to increase the growth rate of the pigs.

**11.** Use according to claim 9 or 10, wherein said glycine compound is used to reduce said feed conversion rate.

**12.** Use according to any one of the claims 9 to 11, wherein said glycine compound is added to a feed for pigs.

**13.** Use according to any one of the claims 9 to 12, wherein said pigs are male pigs.

**Patentansprüche**

**1.** Ein Verfahren zur nicht-therapeutischen Behandlung von Schweinen zur Senkung der Futterverwertungsrate des Futtermittels zur Ernährung der Schweine und/oder zur Steigerung der Wachstumsrate der Schweine, wobei die Behandlung die orale Verabreichung von zumindest einer Glycinverbindung an die Schweine umfasst, wobei die Glycinverbindung aus N,N-Dimethylglycin (DMG) oder einem Salz davon ausgewählt wird.

**2.** Das Verfahren nach Anspruch 1, wobei die Glycinverbindung über das Trinkwasser der Schweine verabreicht wird.

**3.** Das Verfahren nach Anspruch 1 oder 2, wobei die Glycinverbindung über das erwähnte Futtermittel verabreicht wird.

**4.** Das Verfahren nach irgendeinem der Ansprüche 1 bis 3, wobei die Glycinverbindung in einer Menge von zwischen 0,001 und 0,5 Trockengewichtsprozent des erwähnten Futtermittels verabreicht wird, vorzugsweise in einer Menge von zwischen 0,005 und 0,1 Trockengewichtsprozent des erwähnten Futtermittels und noch besser in einer Menge von zwischen 0,01 und 0,08 Trockengewichtsprozent des erwähnten Futtermittels.

**5.** Das Verfahren nach irgendeinem der Ansprüche 1 bis 4, wobei die Glycinverbindung männlichen Schweinen oral verabreicht wird.

**6.** Das Verfahren nach irgendeinem der Ansprüche 1 bis 5, wobei die Schweine entwöhnte Ferkel oder Mastschweine umfassen und die Glycinverbindung vorzugsweise im Futtermittel enthalten ist, mit dem die Schweine gefüttert werden.

**7.** Das Verfahren nach Anspruch 6, **dadurch gekennzeichnet, dass** die erwähnten Schweine zwischen 21 Tage und 7 Monate alt sind.

**8.** Das Verfahren nach irgendeinem der Ansprüche 1 bis 4, wobei die Schweine trächtige oder säugende Sauen umfassen, wobei die Futterverwertungsrate des Futtermittels unter Berücksichtigung des Gewichts der Sauen und, für säugende Sauen, das Gewicht der Sauen und ihrer Ferkel bestimmt wird, und die Glycinverbindung vorzugsweise in einem Geburtsfutter oder in einem Laktationsfutter enthalten ist, mit dem die Sauen gefüttert werden.

**9.** Verwendung einer Glycinverbindung zur Senkung der Futterverwertungsrate von Futtermittel zur Ernährung von Schweinen und/oder zur Steigerung der Wachstumsrate von Schweinen, wobei die Glycinverbindung aus N,N-Dimethylglycin (DMG) oder einem Salz davon ausgewählt wird.

**10.** Verwendung nach Anspruch 9, wobei die erwähnte Glycinverbindung verwendet wird, um die Wachstumsrate der Schweine zu steigern.

11. Verwendung nach Anspruch 9 oder 10, wobei die erwähnte Glycinverbindung verwendet wird, um die erwähnte Futterverwertungsrate zu senken.

12. Verwendung nach irgendeinem der Ansprüche 9 bis 11, wobei die erwähnte Glycinverbindung einem Futtermittel für Schweine zugesetzt wird.

13. Verwendung nach irgendeinem der Ansprüche 9 bis 12, wobei die erwähnten Schweine männliche Schweine sind.

## Revendications

1. Procédé de traitement non thérapeutique de porcs dans le but de réduire le taux de conversion de l'alimentation utilisée pour nourrir les porcs et/ou pour accroître le taux de croissance des porcs, lequel traitement comprend l'administration par voie orale d'au moins un composé de glycine aux porcs, lequel composé de glycine est sélectionné parmi la N,N-diméthylglycine (DMG) ou un de ses sels.

2. Procédé selon la revendication 1, dans lequel le composé de glycine est administré aux porcs par l'intermédiaire de l'eau potable.

3. Procédé selon la revendication 1 ou 2, dans lequel le composé de glycine est administré par l'intermédiaire de ladite alimentation.

4. Procédé selon l'une quelconque des revendications 1 à 3, dans lequel le composé de glycine est administré dans une quantité comprise entre 0,001 et 0,5 % en poids sec de ladite alimentation, de préférence dans une quantité comprise entre 0,005 et 0,1 % en poids sec de ladite alimentation et mieux encore dans une quantité comprise entre 0,01 et 0,08 % en poids sec de ladite alimentation.

5. Procédé selon l'une quelconque des revendications 1 à 4, dans lequel le composé de glycine est administré par voie orale aux porcs mâles.

6. Procédé selon l'une quelconque des revendications 1 à 5, dans lequel les porcs comprennent des porcelets sevrés ou des porcs en engraissement, le composé de glycine étant de préférence inclus dans ladite alimentation qui est donnée aux porcs.

7. Procédé selon la revendication 6, **caractérisé en ce que** lesdits porcs ont entre 21 jours et sept mois.

8. Procédé selon l'une quelconque des revendications 1 à 4, dans lequel les porcs comprennent des truies en gestation ou en lactation, le taux de conversion de l'alimentation étant déterminé en tenant compte du poids des truies et, pour les truies en lactation, du poids des truies et de leurs porcelets, et le composé de glycine étant de préférence inclus dans une alimentation de parturition ou dans une alimentation de lactation qui est donnée aux truies.

9. Utilisation d'un composé de glycine pour réduire le taux de conversion de l'alimentation utilisée pour nourrir des porcs et /ou pour accroître le taux de croissance de porcs, lequel composé de glycine est sélectionné parmi la N,N-diméthylglycine (DMG) ou un de ses sels.

10. Utilisation selon la revendication 9, dans laquelle ledit composé de glycine est utilisé pour accroître le taux de croissance des porcs.

11. Utilisation selon la revendication 9 ou 10, dans laquelle ledit composé de glycine est utilisé pour réduire ledit taux de conversion de l'alimentation.

12. Utilisation selon l'une quelconque des revendications 9 à 11, dans laquelle ledit composé de glycine est ajouté à une alimentation pour porcs.

13. Utilisation selon l'une quelconque des revendications 9 à 12, dans laquelle lesdits porcs sont des porcs mâles.

EP 2 207 432 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 3907869 A
- WO 9904784 A

### Non-patent literature cited in the description

- **KREBS E.T. ; BEORD N.H. ; MALIN R.** *Int. Red. Med.,* 1954, vol. 164, 18
- **NIZAMETIDINOVA, G.** Reports of the Kazan Veterinary Institute. *Reports of the Kazan Veterinary Institute,* 1972, vol. 112, 100-104
- **ROGER V. KENDALL ; JOHN W. LAWSON.** *Recent Findings on N, N-Dimethylglycine (DMG): A new Nutrient for the New Millenium,* 2000
- **KALMAR I.D. et al.** Effect of dimethylglycine on performance and ascites-related parameters in broiler chickens. *the abstract of their presentation on the 9th ESVCN Congress,* 22 September 2005
- **FEKETE S. ; EGEDÜS M. ; SÓS E.** *Magyar Állatorvosok Lapja,* 1978, vol. 34 (5), 311-314
- Nutrient Requirements of Swine. National Research Council, 1988
- **NOBLET J ; VAN MILGEN J.** Energy value of pig feeds: Effect of pig body weight and energy evaluation system. *J. Anim Sci.,* 2004, vol. 82, E229-E238